# EUROPEAN PATENT APPLICATION

(11) **EP 2 168 542 A1**
(43) Date of publication of application: **31.03.2010**
(21) Application number: 08165270.3
(22) Date of filing: 26.09.2008
(51) Int. Cl.: A61F 13/56, A61F 13/551

(54) **Feminine hygiene article with visual indicator**

(71) Applicant: The Procter and Gamble Company, Cincinnati, Ohio 45202 (US)
(72) Inventor: Gagliardi, Ivano, 65123, Pescara (IT); Veglio, Paolo A, 65129, Pescara (IT); Branca, Andrea, 65124, Pescara (IT); Breda, Sandro, 65100, Pescara (IT)
(74) Representative: L'Huillier, Florent Charles

(57) **Abstract**

A feminine hygiene article (10) having a longitudinal axis (L), a transverse axis (T) and a periphery. The article comprises a backsheet (16) and a topsheet (12). The backsheet (16) has a body-facing side and a garment-facing side, wherein the garment-facing side comprises an adhesive area (24) and a non-adhesive area (22) adjacent to the periphery of the article. The article further comprises a release cover (18) releasably attached to the adhesive area (24). The article comprises a visual indicator (20), wherein said visual indicator is disposed to indicate to the user the position of the non-adhesive area (22). The visual indicator is not disposed on the release cover (18).

## Description

### FIELD OF THE INVENTION

This invention relates to feminine hygiene articles.

### BACKGROUND OF THE INVENTION

The feminine hygiene articles of the invention are of the type which is placed by the user in her undergarment and comprise articles commonly referred to as sanitary towels (or sanitary pads), light adult incontinence absorbent articles and pantiliners. Whereas sanitary towels are generally used to collect relatively large flows of liquid (menses), pantiliners are relatively thin feminine hygiene articles designed to protect the user's underwear and clothing from soiling by light vaginal discharges. For example, many women have developed the habit of wearing a pantyliner between their menstrual periods to protect their clothing from bodily fluids such as vaginal discharges and slight urinary leak.

In general, feminine hygiene articles comprise an impermeable backsheet forming the garment-facing side of the article, a topsheet forming the opposite, body-facing side of the article and an absorbent structure, normally referred to as an absorbent core, placed in-between. The body-facing side of the article is the first exposed to the flow of the bodily fluid, which is then absorbed and directed to the absorbent structure. The fluid impermeable layer prevents the fluid from leaking from the absorbent structure to the garment through the garment-facing side of the article.

W02005/115296 discloses absorbent articles having indicator markers disposed to indicate proper alignment of the absorbent article with respect to undergarment-specific features such as the curved leg opening.

Feminine hygiene articles usually comprise on their garment-facing side an adhesive area which is designed to stick to the undergarment, so that the article remains in position during wear. The adhesive area may cover entirely the garment-facing side of the article, or the garment-facing side may also comprise a non-adhesive area. Before use, the adhesive area is normally protected by a release cover. The release cover may be a release paper which does not extend beyond the periphery of the article, or may be a wrapper sheet which extends beyond the periphery of the article.

It has been proposed to facilitate the removal of the release cover by placing a so-called "finger lift" on one end of the article. For example, a relatively small area of the garment-facing side of the backsheet that is easily accessible by the user (normally at the periphery of the article, and in particular at its longitudinal end) may be non-adhesive. The user can easily grasp this non-adhesive area since it is not attached to the release cover, and use it to initiate the removal of the release cover from the article by pulling release cover and article apart. It has also been proposed to provide release liners with a notch corresponding to a non-adhesive area, see for example WO03/072004 "Absorbent article including undergarment fastener adhesive having improved adhesive pattern".

Some pantiliner products have been proposed with a release paper comprising a printed signal indicating the position of the finger lift. This signal was found useful for indicating to the user the position of the finger lift before separating the release cover. However the present inventors have come to the insight that once the absorbent product is placed in the user's undergarment, it is practically impossible for the user to recognize visually where the finger lift is positioned. If the user wishes to use the finger lift as removal aid for the soiled article, she may thus have to check different areas of the soiled article until finding the position of the finger lift. The present inventors have come to the insight that this yet unrecognized problem could be solved by the invention as described hereinbelow.

### SUMMARY OF THE INVENTION

The present invention relates to a feminine hygiene article having a longitudinal axis, a transverse axis and a periphery. The article comprises a backsheet and a topsheet. The backsheet has a body-facing side and a garment-facing side, wherein the garment-facing side of the backsheet comprises an adhesive area and a non adhesive area. The article may comprise an absorbent core. The article further comprises a release cover releasably attached to the adhesive area of the garment-facing side of the backsheet. The article further comprises a visual indicator which is disposed to indicate to the user the position of said non-adhesive area. The visual indicator is not disposed on the release cover. In other words, the visual indicator is disposed on a portion of the article other than the release cover.

The visual indicator may be a printed area corresponding at least partially to said non-adhesive area. The printed area may for example be printed on the body-facing side of the backsheet or on the garment-facing side of the backsheet. The printed area may be uniformly printed or not.

The visual indicator may also be printed on any layers of the article, other than the release cover. The visual indicator may also be disposed partially or in whole in an area outside of the non-adhesive area, as long as it provides a visual indication to the user of the position of the non-adhesive area.

Because the visual indicator is not disposed on the release cover, it will remain on the article when the article is placed on the user's undergarment and can therefore facilitate the removal of the soiled article from the undergarment.

### BRIEF DESCRIPTION OF THE DRAWINGS

While the specification concludes with claims which particularly point out and distinctly claim the invention, it is believed the present invention will be better understood from the following description of preferred embodiments taken in conjunction with the accompanying drawings, in which like reference numerals identify identical elements and in which:
Fig. 1 is a perspective view of an embodiment of the present invention;
Fig. 2 is an exploded perspective view of the pantiliner of Fig. 1;
Fig. 3 is an exploded perspective view of the pantiliner of Fig. 1 from another angle;
Fig. 4 is a perspective view of the pantiliner of Fig. 1 with the release cover in the process from being separated from the backsheet;
Fig. 5 is a plan view of the pantiliner of Fig. 1 placed in an undergarment;
Fig. 6 is a plan view of another embodiment of the invention;
Fig. 7 is a plan view of another embodiment of the invention;
Fig. 8 is a plan view of another embodiment of the invention;
Fig. 9 is a plan view of another embodiment of the invention;
Fig. 10 is a plan view of an embodiment of the invention where the release cover is a wrapper sheet and is opened;
Fig. 11 is a view of the embodiment of Fig.10 with the wrapper sheet partially opened.

### DETAILED DESCRIPTION OF THE INVENTION

### Definitions

Unless explicitly stated otherwise, the term "comprising" is to be construed as open ended, meaning that other features, steps or ingredients can be added as long as they are suitable to be used in a feminine hygiene article. The expressions "... comprising a ..." and "... comprises a ..." are thus to be construed as "... comprising one or more ..." or "... comprises one or more ..." respectively.

The term "feminine hygiene articles" refers to the type of absorbent hygiene articles externally worn by women, usually for menstrual and/or light incontinence control. These articles are commonly referred to as pads, pantiliners, liners, sanitary napkins or sanitary towels. These articles have usually a generally flat surface and are typically held in place adjacent the user's crotch (i.e. the pubic region) by the user's undergarment on which they are affixed via adhesive or other joining means.

The term "body-facing side" refers to the side of the absorbent article or a layer thereof facing the body of the user when in use. The "garment-facing side" is the opposite side of the article or layer.

The term "longitudinal axis" refers to the imaginary line centered between the longitudinal side edges of the article and which is generally aligned with the vertical plane which bisects a standing wearer into left and right body halves. The longitudinal axis is represented by the line L on the drawings. The longitudinal axis conceptually divides the article in two halves. By conceptually we mean that the longitudinal axis is normally not physically present or visible in the article.

The term "transversal axis" refers to the imaginary line which is perpendicular to the longitudinal axis in the plane of the body-facing side of the article and intersects it in the middle of the length of the article as measured on the longitudinal axis. The transversal axis conceptually divides the article in two halves. By conceptually we mean that the transversal axis is normally not physically present or visible in the article.

The term "periphery" refers to the boundary of the article in the horizontal plane.

### Absorbent article 10

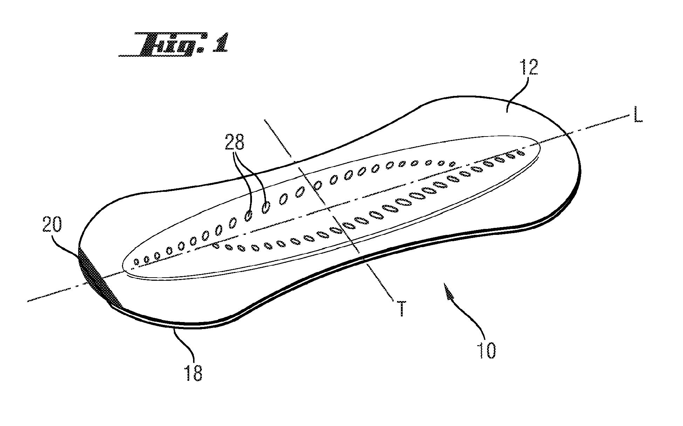

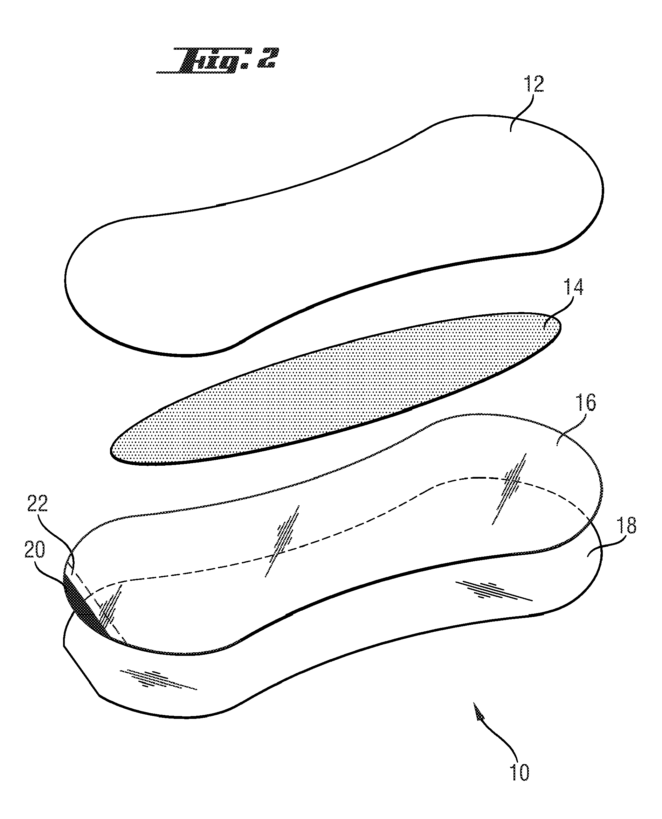

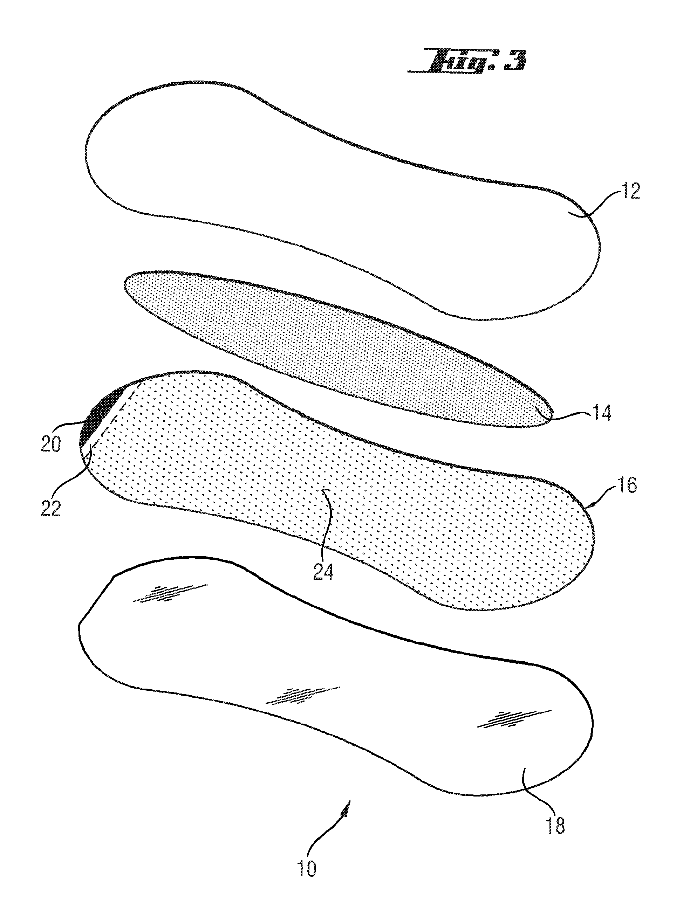

Referring now to Figures 1, a feminine hygiene article 10 according to the invention is depicted in a perspective view. The same article is represented in an exploded view on Fig. 2 and 3. The article shown comprises, from top to bottom, the following layers: a topsheet 12, an absorbent core 14, and a backsheet 16. A release cover 18 is releasably attached to the adhesive area 24 of the garment-facing side of the backsheet layer.

The release cover 18 may be discarded by the user when the absorbent article is placed in the undergarment. The release cover is also discarded for the purpose of measuring the thickness of the article.

In the embodiment of Fig.1, the visual indicator 20 is a printed area 20 of the backsheet 16 corresponding partially to the non-adhesive area 22 of the garment-facing side of the article. The adhesive area 24, in the embodiment of Fig. 1, covers the majority of the surface of the garment-facing side of the backsheet, but other configurations are possible.

As shown in the embodiment of Fig.1, the visual indicator 20 may be printed on the backsheet 16 and still be visible by transparency on the body-facing side of the article through the topsheet 12. The visual indicator may provide two advantages. First, it highlights the position of the finger lift area, which is the area 22 of the garment-facing side of the backsheet which is non-adhesive and which can be used to easily separate the release cover 18 from the article. Second, when the article is placed in the undergarment, the visual indicator 20 indicates to the user the position of the non-adhesive area 18 and allows the user to grasp this end of the article which is not attached to the undergarment, thus facilitating the removal of the soiled article. The different components of the article of the invention briefly described above will now be discussed in more details.

### Topsheet 12

The topsheet 12 is the layer of the article which is oriented towards and contacts the body of the wearer, and is therefore the first layer to receive the bodily discharges. The topsheet is normally made of a single layer, as represented in the Figures, but may also comprises more than one layer (for example a central topsheet layer and two overlapping lateral stripes, as disclosed in WO93/09744 or EP766,953).

The topsheet 12 is normally liquid pervious. The term "liquid pervious" as used herein refers to components that allow liquids to pass therethrough without significantly retarding or obstructing the transmission of such liquids therethrough.

It is envisaged that any conventional topsheet materials may be used within the invention. Advantageously, the topsheet may not be opaque so that a visual indicator may be disposed on a layer underlying the topsheet and still be visible by a user looking upon the body-facing side of the topsheet 12. Suitable topsheets may be made for example from nonwoven materials or perforated polyolefinic films. An exemplary topsheet suitable for use herein is a relatively hydrophobic 20 gsm spunbonded nonwoven web comprising bicomponent fibers of the sheath core type (PP/PE), exemplarily available from Pegas a.s., Czech Republic, under the tradename 101800000200-WW/ZZ.

If desired, the topsheet 12 may be treated with a surfactant to enhance liquid penetration to the core. The surfactant is typically non-ionic and should be nonirritating to the skin. A surfactant density of about 0.01 milligrams per square centimeter of topsheet area is normally suitable. An exemplary surfactant is sold by the Glyco Chemical, Inc. of Greenwich, Connecticut as Pegosperse 200 ML. The topsheet may have a plurality of apertures to permit liquids deposited thereon to pass through to the core more quickly.

The topsheet 12 and the backsheet 16 are preferentially peripherally joined using known techniques such as heat embossing. The layers may also be glued to each other. The topsheet 12 may be contiguous with the backsheet 16 with these two layers forming the periphery of the article.

### Absorbent core 14

The articles of the invention may comprise an absorbent core 14 placed between the topsheet 12 and the backsheet 16. As used herein, the term "absorbent core" refers to a material or combination of materials suitable for absorbing, distributing, and storing fluids such as urine, blood, menses, and/or other body exudates.

The size and shape of the absorbent core 14 may be such that the surface of the core in the horizontal plane is substantially smaller than the surface of the topsheet. By "substantially smaller", we mean that the surface of the core 14 is at least about 10% smaller than the surface of the topsheet 12, or at least about 25% smaller than the surface of the topsheet 12. The absorbent core 14 may be generally centered in the middle of the article. The absorbent core 14 may be disposed away from the periphery of the article to provide improved flexibility along the edges of the article.

By providing an absorbent core having a substantially smaller surface than the topsheet, several benefits may be achieved. The amount of core material used is reduced, lowering the overall costs of manufacturing the product. A core having a smaller surface also increases the overall flexibility of the product, because the regions of the product not provided with a core are generally less rigid than the region where the core is situated. The visual indicator may be advantageously disposed in an area which is not directly situated under the core, because the core may be opaque or of a limited transparency. On the other hand, if the article comprises a core which has substantially the same surface as the article, as is usually the case for sanitary napkins, it may be advantageous to place the visual indicator above the core so that it is clearly visible on the topsheet of the article.

The absorbent core 14 may have an oval shape as represented in the figures but may also have any other shape. For example it is typical for absorbent cores to be rectangularly shaped for ease of manufacturing. However flexibility may be better with cores having a curved shape and not comprising right angles.

The absorbent core 14 can be made of any suitable materials. Nonlimiting examples of suitable liquid-absorbent materials include comminuted wood pulp which is generally referred to as airfelt; creped cellulose wadding; absorbent gelling materials including superabsorbent polymers such as hydrogel-forming polymeric gelling agents; chemically stiffened, modified, or cross-linked cellulose fibers; meltblown polymers including co-form; synthetic fibers including crimped polyester fibers; tissue including tissue wraps and tissue laminates; capillary channel fibers; absorbent foams; absorbent sponges; synthetic staple fibers; peat moss; or any equivalent material; or combinations thereof. The absorbent core comprise superabsorbent polymer (SAP), normally distributed within a matrix of cellulosic fibers, for example in order to reduce the thickness of the absorbent core.

The absorbent core may be unitary, or may be a laminate of two or more layers. For example, the core may comprise a fluid impermeable barrier layer (e.g. a PE Patch) on its backsheet-facing side to prevent fluids retained by the absorbent core from striking through the pantiliner and soiling adjacent garments. An exemplary PE patch is a 25 gsm poly film available from Britton Taco (UK) under trade name ST-012A-White.

Further generic information regarding absorbent cores can be found in prior patent publications, see for example WO0207662A1 and W09119471.

### Backsheet 16

The general function of the backsheet is to prevent discharges absorbed by the core from escaping the feminine hygiene article and soiling the clothing of the wearer. The backsheet 16 may be made of any suitable material in particular any standard backsheet materials. These materials are generally flexible, liquid resistant, and liquid impervious.

Any conventional backsheet materials may be used within the invention, such as polyolefinic films or nonwoven webs. Nonwoven webs may be advantageous because they normally provide better breathability for the articles and may be cheaper than polyolefinic films. For example, a relatively hydrophobic 18 grams per square meter (gsm) spunbonded nonwoven web of 2 denier polypropylene fibers, exemplarily available from Pegas a.s. Czech Republic under the trade name 121801000100 may be used. The backsheet may also be a laminate as is know in the art.

The backsheet 16 has a garment-facing side and an opposite body-facing side. The garment-facing side of the backsheet comprises a non-adhesive area 22 and an adhesive area 24. The adhesive area 24 may be provided by any conventional means. Pressure sensitive adhesives have been commonly found to work well for this purpose.

Full adhesive coverage of the backsheet may be applied except for a relatively small non-adhesive area 22 serving as finger lift, as exemplarily shown on Fig. 3. It is known that providing a full adhesive coverage of the garment-facing side of the backsheet except for a non adhesive area 22 may be advantageous to provide a better adherence of the article to the undergarment. It was found however by the inventor that the user may have problem finding the non-adhesive area as when is relatively small, so that the visual indicator of invention is particularly useful in the context of a "full" adhesive coverage where the non-adhesive area is relatively small and/or when the article comprises only one (a single) non-adhesive zone, for example when the total non-adhesive area represents from about 0.5% to about 15% of the total surface of the backsheet, or even less about 10% of the total surface of the backsheet.

The shape of the adhesive area may also be different, for example it is also common to use one or two or more strips of longitudinally oriented adhesive strips instead of full coverage. The adhesive strips may then be continuous or intermittent. For example two longitudinally oriented strips, one on each side of the longitudinal axis, may be applied. The adhesive may be applied via direct slot coating application process. Mechanical adhesive means may be also provided, such as microscopic hooks placed on the backsheet and designed to attach to the fibers present in some undergarments, as in a hook-and-loop fastener. If the article comprises wings, these will normally also comprise on their garment-facing side an adhesive area.

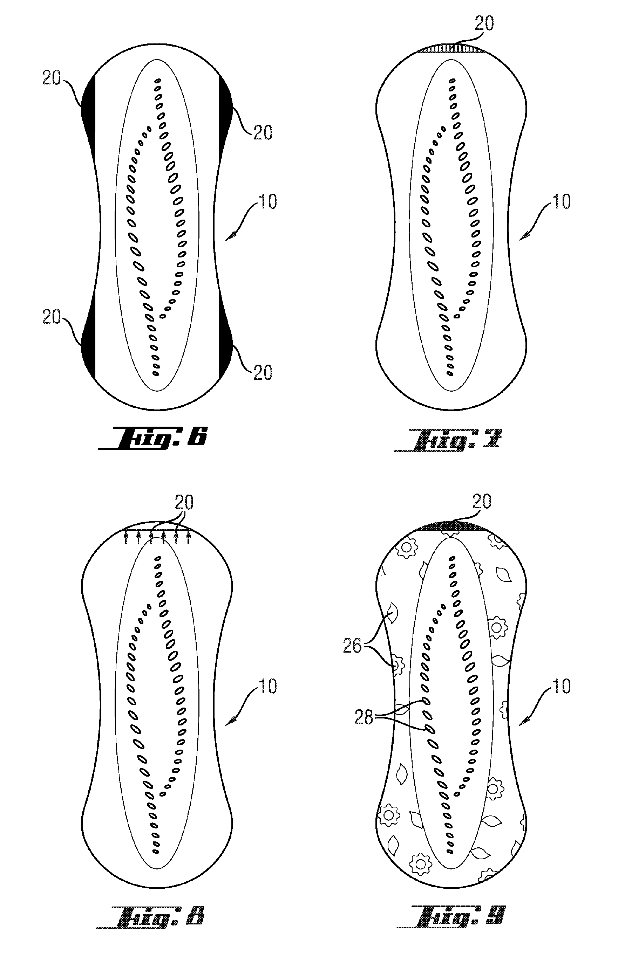

The garment-facing side of the backsheet 16 comprises a non-adhesive area 22 which is placed adjacent to the periphery of the article. This non-adhesive area 22 may intersect the longitudinal axis L of the article. The non-adhesive area 22 may be used as a so called "finger lift", to ease the removal of the releasable cover from the release cover 18 before use or from the undergarment post-use. The article may also comprise several discrete non-adhesive areas, each placed adjacent to the periphery of the article, which may each have a visual indicator, as shown on Fig. 6.

### Visual indicator 20

The feminine hygiene article of the invention comprises a visual indicator 20 disposed to indicate to the user the position of the non-adhesive area. As used herein, the term "visual indicator" designates a feature providing a visual contrast with the rest of the article. The visual indicator can be recognized by the user of the article as providing an indication of the position of the non-adhesive area. For example, the visual indicator may be a printed area which may correspond partially or entirely to the non-adhesive area 22 of the garment-facing side of the backsheet. By "correspond" we mean placed on the area itself or on an area of any other layer of the article directly overlapping the area. It may also be envisaged to place the visual indicator in an area outside the non-adhesive area, as is shown on Fig. 8.

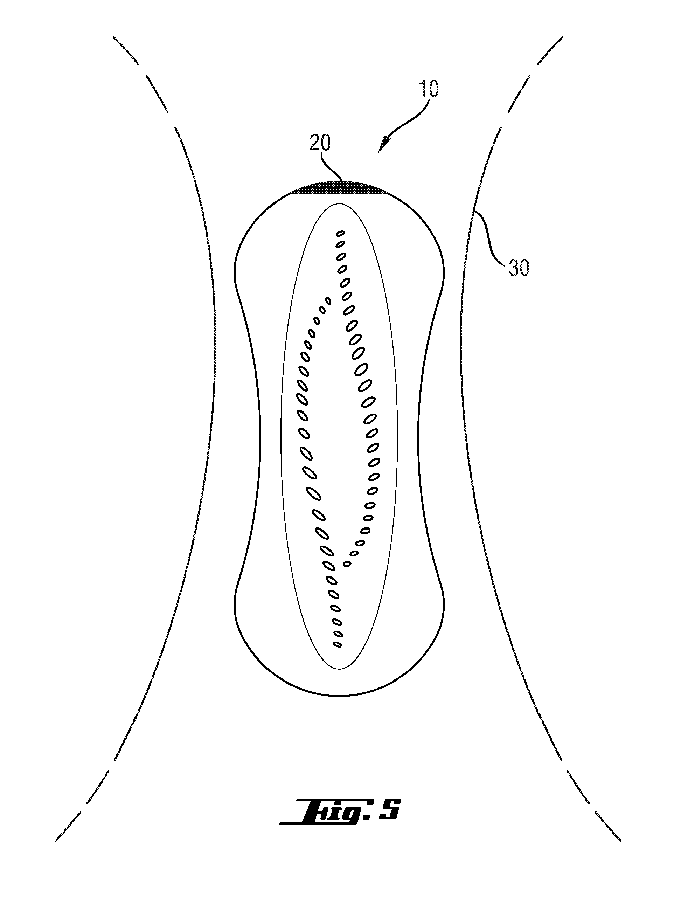

The visual indicator is not disposed on the release cover, or in other words is disposed on a component which is not the release cover, so that it remains on the article even after the article is placed on the user's undergarment 30, as exemplarily shown on Fig. 5. This may facilitate the removal of the soiled article by the user.

The visual indicator 20 may be a printed area or may be provided by other means of providing a visual contrast, for example a piece of colored or pigmented material may be used to provide a distinctive colour, or a specific embossment, fold or pleat pattern may be provided to also provide a visual contrast. Heat crimping may also form a distinctive pattern. However, a printed area may be advantageous because it may be less costly and complex to produce than other means.

The visual indicator 20 is advantageously visible on the body-facing side of the article. By "visible on the body-facing side of the article", we mean that a subject having a good vision in both eyes (10/10) can recognize the presence of the visual indicator 20 when the article is placed on a white background at a distance of 50 cm in a brightly lit room with incandescent light (the article being placed flat as during in use condition). The visual indicator may be disposed on the topsheet or on an underlying layer such as the backsheet, in which case the visual indicator should advantageously be visible by transparency on the body-facing side of the article. The visual indicator is advantageously visible on the body-facing side of the article with and without the release cover. In this way, the release cover can be more easily detached because the position of the non-adhesive area is indicated to the user at the point of use, as well as at the point where the user seeks to detach the used product from the undergarment.

Conventional topsheet and backsheet layers are normally substantially transparent or translucent so that a printed pattern will normally be visible on the body-facing side of the article by transparency even if the visual indicator is not directly placed on the topsheet. Core materials are usually less transparent, so that it may be advantageous that the visual indicator be disposed at least partially outside the area corresponding to the core, or alternatively on the body-facing side of the core or a layer situated above the core. For articles with so called "full surface core design" where the absorbent core shape corresponds substantially to the product shape, the presence of the visual indicator may also help preventing the accidental product delamination by the user both at the release paper removal step and at the panty removal step after use. This advantage is particularly effective in presence of ticker/bulkier core where the inter-core delamination sometimes occur when the user mistakenly confuses the edge of the core with the edge of the article.

The visual indicator 20 may be disposed on a relatively small surface compared to the overall surface of the article. This is because the non-adhesive area 22 corresponding to a finger lift is normally also relatively small. The surface of the visual indicator may for example represent from about 0.5% to about 15% of the total surface of the article. Advantageously, the visual indicator may have a surface coverage of between about 1% and about 10%, or between about 2% to about 7.5% of the overall surface of the article. As indicated above, the visual indicator may be in the form of a uniformly printed area or may be provided by other means.

In practice, it may be difficult to apply the visual indicator so that is corresponds exactly with the non-adhesive area 22 due to manufacturing constraint of high-speed production. The visual indicator may be disposed to cover a smaller surface area than the non-adhesive area, as shown on Fig. 2 and 3, or a larger surface area if wished. In the embodiment represented on Fig.1, the width of the printed area 20 in the longitudinal direction is about 7mm and the width of the non-adhesive area in the same direction is about 9mm measured from the longitudinal end of the article. On the other hand, as indicated above, it may also be envisaged that the visual indicator be provided by leaving an area corresponding to the non-adhesive area white, while printing the rest of the layer uniformly or with a relatively dense pattern, the visual indicator being thus formed by contrast, although in practice this may be relatively more costly than printing the visual indicator.

More than one visual indicator may be provided, for example where several discrete non-adhesive zones are provided as finger lift zones, as exemplary shown on Fig.6 where the printed area 20 corresponds approximately to the non-adhesive zones.

The visual indicator may also be non-uniformly applied, as shown on Fig. 7 for example, where a series of stripes is placed instead of a uniform zone. The visual indicator may also be placed on an area outside the non-adhesive zone, as long as it still clearly indicates the position of the non-adhesive zone to the user, for example via a sign such as one or more arrows, as represented on Fig. 8.

If the visual indicator 20 is provided by a printed area, it may be printed by any conventional printing method, such as flexo print or roto gravure printing. The intensity of the ink should be sufficiently strong so that the visual indicator is visible on the body-facing side of the article, in particular through at least a portion of the topsheet 20 if the printed area is printed on underlying layer such as the backsheet. A black ink may be used, but it may be preferred to use a coloured ink such as pink, violet, green, purple, blue or yellow, or even a combination of different coloured inks. If the visual indicator is provided by a uniformly printed zone (as exemplarily represented on Fig.1), it may be advantageous to have a minimum color difference between the printed zone and the rest of the article as measured with the ΔE* parameter. The significance and practical consideration on how to measure the ΔE* value can be found for example in US7,241,280B2, col.4 line 58 to col.9 line 12. The color difference between the printed area and the rest of the article as measured in the center of article, i.e. the intersection of the longitudinal and transversal axis, may advantageously be at least 3.5 (ΔE* ≥ 3.5). In some cases, the printed area may be too small for the size of the spectrophotometer probe indicated in US7,241,280B2, in that case a smaller probe may be used or a larger sample zone constructed from cut-out sections of several articles.

As indicated above, the visual indicator may advantageously be printed on a layer of the article, for example the backsheet. The layer which is printed may be printed "off-line" or "on-line". By "off-line", we mean that the material used for the layer will not be printed on the manufacturing line where the different layers are assembled, but at a different location and in a separate step, for example at the supplier's factory. On the other hand, the area may also be printed "in-line", which means that a printing device is placed on the converting line and will be printing the material as it is unrolled and usually before it is assembled with the other layers. Both off-line and on-line printing are known in the art.

### Release cover 18

The article comprises a release cover 18 releasably attached to the adhesive area 24 of the garment-facing side of the backsheet. The release cover is removed by the user before placing the article in the undergarment. Different types of release covers exist, in particular so-called release paper, one-piece wrapper sheet and two-piece wrapper sheet. Any conventional release covers may be used with the present invention.

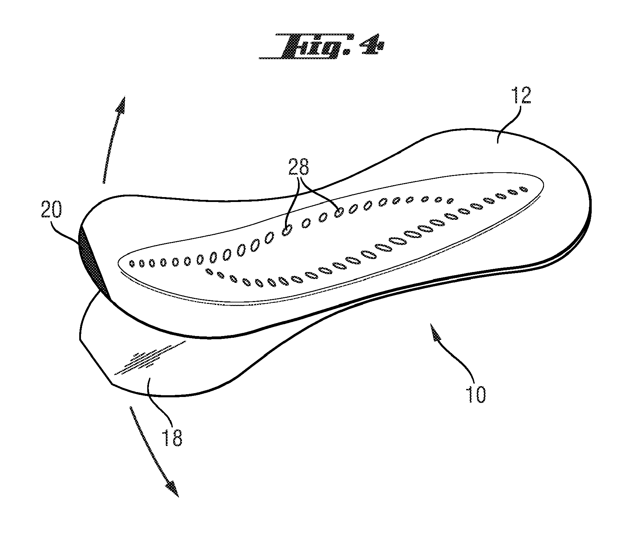

Release papers are normally used for articles that are provided flat (not folded) to the user and may be of about the same size (or smaller) as the article, as shown on Fig. 1 to 9. The release papers may be made of a paper material, one side of which has been treated with silicone and this siliconized layer may be releasably attached to the adhesive area. Sometimes a non-woven material is used in place of a paper material, but the principle and use remains the same. The term release paper has used herein will therefore refer to all release covers which are not a wrapper sheet (see below), in particular release covers used for articles sold in a non-folded configuration and/or which do not extend beyond the periphery of the article. The release paper may also comprise a small cut-out section in an area corresponding to the non-adhesive area, in order to further help the removal of the release paper from the backsheet.

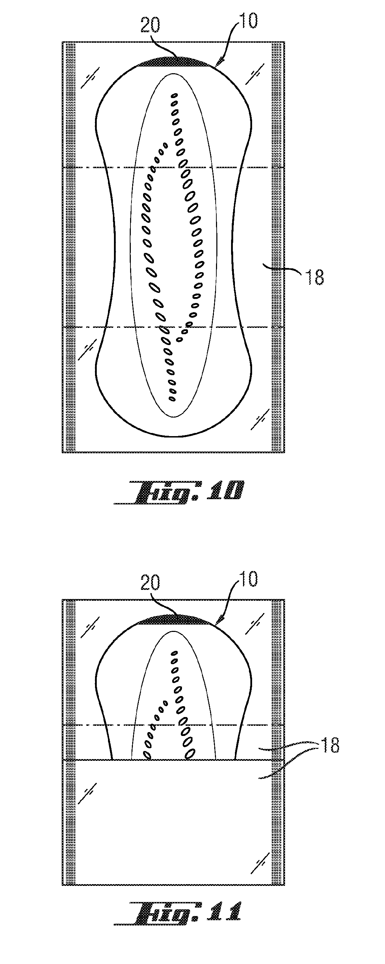

The term "wrapper sheet" refers to the type of release covers which extend beyond the periphery of the article and are used to wrap the article before use, as shown on Fig. 10 - 11. A one-piece wrapper sheet can be used, which as the name indicates, refers to a single piece of material, usually a plastic material siliconized on one side. A two-piece wrapper sheet may also be used, in which a release paper is permanently glued to a larger wrapper sheet, the article being releasably attached to release paper. The articles is usually folded and enclosed by the wrapper sheet (one-piece or two-piece) which can be sealed on its sides.

These various types of release covers are known in the art. Suitable examples include BL 30 MG-A SILOX EI/O, BL 30 MG-A SILOX 4 P/O available from Akrosil Corporation, and M&W films available from Gronau in Germany.

### Printing pattern 26

If the visual indicator is obtained by a printing step, a decorative pattern 26 may also be printed of the rest of the layer to provide an attractive appearance to the article. As represented on Fig. 9 for example, the article may also comprise a pattern 26 printed on the rest of the backsheet. The visual indicator contrasts with the printed pattern 26 so it is clearly identifiable even in the presence of a further decorative pattern.

This printed pattern 26 may comprise, as represented in Fig. 9, a series of dispersed discrete printed elements which may be identical or differ within the printed pattern 26. Of course, other discrete elements than those represented may be used, for example floral decorative elements (flowers, leaves), stars, or any other decorative elements. The printed pattern 26 may also consist of a continuous element rather than discrete elements, as long as the visual indicator remains recognizable.

### Embossed pattern 28

The articles of the invention may further present an embossed pattern 28. Embossing may normally serve several functions, such as providing a bonding between overlying layers and/or providing a quicker fluid pathways through the topsheet towards the core. Ideally, the embossing may also be aesthetically pleasing. The embossed pattern can be achieved with standard techniques such as thermal bond, ultrasonic bond or pressure. A suitable process is thermal bonding wherein the layers are passed through two steel rolls where one is engraved with the visual pattern and the other is flat. Both rolls are warmed to temperature suitable to melt the layer (typical range from 90 to 170°C). The embossed pattern may be partially or entirely comprised within the area corresponding to the core.

The embossing roll may be engraved using conventional techniques such as machine tooling for most embossed patterns, but it may be preferred to use acid etching or laser engraving to provide a finer engraving, and thus a finer embossed pattern.

### General

The thickness of the absorbent articles according to the invention may be typical for the type of applications foreseen. For pantiliners (as exemplarily represented in the Figures), the thickness is generally thinner as for sanitary pads for examples. The articles of the invention may have a relatively small thickness (also called "caliper") of less than about 5 millimeters, as measured using the standard test described below. The articles may be even thinner, to provide very discrete articles. The lowest limit for the thickness of the article will be dictated by technical feasibility, but obtaining an effective article thinner than 0.4 mm may be difficult. Suitable thickness ranges for the articles of the invention include of from about 0.4 mm to about 4 mm, and from about 0.6 mm to about 3 mm. As used herein, the term "thickness of the article" refers to the thickness value measured in the center of the article, i.e. normally including the thickness of the core 14.

The dimensions of the articles of the invention in the horizontal plane are typical of the articles in the field. For example the length of such products will normally lie in a range of from about 8 cm to about 20 cm for the length of the article, and of from about 3 to about 9 cm for its width. The overall surface of the article (so typically the surface of the topsheet) may also lie within the usual range found for these articles, which normally would be of from about 40 cm² to about 250 cm². For the purpose of providing exemplary dimensions of a pantiliner such as the one represented in the Figures, such a pantiliner may have a length of 15 cm, a width (at center) of 4.8 cm, an overall surface of the article of 79 cm² and a core area surface of 38 cm². An exemplary thickness of the article represented is 0.9 mm.

The article may or may not comprise so-called "wings", which are side-wrapping elements destined to be folded around the undergarment. These wings are however normally used for sanitary pads and are not normally present for thinner products such as pantiliners.

The articles of the invention are normally disposable, i.e. are not intended to be re-usable or washable but are normally disposed of after use.

### Method of manufacture

The sanitary articles of the present invention may be produced industrially by any conventional means. The different layers may thus be assembled using standard means such as embossing (e.g. thermal bonding) or gluing or a combination of both. The converting line may comprise a printed step wherein the ink is applied to the backsheet of the article. It is however also possible to carry the printed step on the backsheet outside the converting line of the article, before this layer is joined with any of the other layers.

### Thickness measurement

The articles of the inventions may be relatively thin and not bulky, so that the thickness measurements will be less dependent of the pressure applied when making the measurement, than for example for bulky articles such as thick pads. The following specific method may be used to measure the thickness of the article of the invention. The equipment may comprise an apparatus capable of measuring thickness with a 0.01 mm tolerance. A commercial supplier of such equipment is for example Ono Sokki (www.onosokki.net), for example their Caliper Gauge GS-503 and digital readout DG 2610 may be used. The caliper gauge is fitted with a foot, which may have an exemplary 24.13 mm diameter. A suitable pressure exerted when the measurement is made is 0.689 kPa.

The test procedure is as follows. Make sure the micrometer is zeroed. Place the article without the release cover on the base plate, the topsheet facing up. If the article was provided in a compressed state (as is sometimes the case in certain packaging), the article is let to rest about 10 mn before its thickness is measured. Similarly, if the article was provided folded, the article is first opened and let about 10 mn to rest in its "flat" shape. Position the article on the base plate so that when the foot is lowered, it is in the center of the article. Let the foot gently lowers itself onto the article at a rate of 5 mm/sec +/- 2 mm/sec. Determine the article caliper by reading the micrometer dial 10 seconds after the foot comes to rest. The shaft and foot should deliver approximately 32 grams of force for a pressure of 0.69 +/- 0.02 kPa to the sample with the above mentioned foot having a diameter of 24.13mm.

## Claims

1. A feminine hygiene article (10) having a longitudinal axis (L), a transverse axis (T) and a periphery,
said article (10) comprising a backsheet (16) and a topsheet (12), wherein said backsheet (16) has a body-facing side and a garment-facing side,
wherein the garment-facing side of the backsheet comprises an adhesive area (24) and a non-adhesive area (22), said non-adhesive area being placed adjacent to the periphery of the article,
said article further comprising a release cover (18) releasably attached to the adhesive area (24) of the garment-facing side of the backsheet,
**characterized in that** said article comprises a visual indicator (20), wherein said visual indicator is disposed to indicate to the user the position of said non-adhesive area (22), wherein said visual indicator is not disposed on the release cover (18).

2. An article according to claim 1 wherein said visual indicator is a printed area (20).

3. An article according to claim 2 wherein said printed area (20) corresponds at least partially to said non-adhesive area (22).

4. An article according to any of the preceding claims wherein the printed area is printed on the body-facing side of the backsheet or the garment-facing side of the backsheet.

5. An article according to any of claims 2 to 4 wherein the printed area is uniformly printed.

6. An article according to any of the preceding claims wherein said non-adhesive area is intersected by the longitudinal axis.

7. An article according to claim 6 comprising a single non-adhesive area on the garment-facing side of the backsheet.

8. An article according to any of the preceding claims wherein the total surface of the non-adhesive area represents between 0.5% and 15% of the total surface of the backsheet.

9. An article according to any of the preceding claims wherein the release cover is a release paper which does not extend beyond the periphery of the article.

10. An article according to any of claims 1 to 8 wherein the release cover is a wrapper sheet which extends beyond the periphery of the article.

11. An article according to any of the preceding claims further comprising an embossed pattern (28).

12. A feminine hygiene article (10) having a longitudinal axis (L), a transverse axis (T) and a periphery, said article (10) comprising a backsheet (16) and a topsheet (12), wherein said backsheet (16) has a body-facing side and a garment-facing side,
wherein the garment-facing side of the backsheet comprises an adhesive area (24) and a non-adhesive area (22), said non-adhesive area being placed adjacent to the periphery of the article,
said article further comprising a release cover (18) releasably attached to the adhesive area (24) of the garment-facing side of the backsheet (16),
**characterized in that** said article comprises a printed area (20) corresponding at least partially to the non-adhesive area (22), preferably wherein said printed area corresponds to an area entirely encompassed by the non-adhesive area, and wherein sais area is printed on the body-facing side or the garment-facing side of the backsheet, and said printed area is visible on the body-facing side of the article.
